# EUROPEAN PATENT SPECIFICATION

(11) **EP 4 018 986 B1**
(45) Date of publication and mention of the grant of the patent: **17.12.2025**
(21) Application number: 20217128.6
(22) Date of filing: 23.12.2020
(51) Int. Cl.: A61F 13/0246, A61F 13/02

(54) **PERFORATED ADHESIVE LAMINATE**
PERFORIERTES KLEBSTOFFLAMINAT
LAMINÉ ADHÉSIF PERFORÉ

(43) Date of publication of application: 29.06.2022
(73) Proprietor: Advanced Silicone Coating, 69330 Pusignan (FR)
(72) Inventor: Lecoeuvre, Jean-Francois, F42290 Sorbier (FR)
(74) Representative: Ter Meer Steinmeister & Partner

(56) References cited:
- US-A1- 2008 319 368
- US-A1- 2019 134 255
- US-A1- 2020 246 192
- US-A1- 2020 323 694

## Description

The invention relates to new laminate showing excellent properties when being adhered to a surface such as the skin. The laminate can for example be used for the preparation of articles such as wound dressings which are used for promoting/supporting the process of the healing of a wound.

A wound can be regarded as the separation of the contiguity of tissues of the skin, wherein this can be combined with loss of substance.

The healing of wounds is based on the ability of the skin to regenerate tissue such as epithelial tissue, connective and supporting tissue. The regeneration of tissue is a complex occurrence of cell activities overlapping with each other, wherein said cell activities promote the healing process step by step. A suitable wound dressing may help to provide a favorable environment for said healing process.

Such wound dressings have to be applied on a surface such as a wound or the skin surrounding said wound. On the one hand, it is essential that the wound dressing adheres well to said surface to prevent an undesired slipping or even removal of the wound dressing. On the other hand, it is also important to ensure that, once a wound dressing has to be removed, for example to change the dressing, the removal of the dressing does not negatively affect the surface (of the skin). **In** fact, the properties of the layer facing the wound appear to be essential to ensure the above-indicated adherence properties of a wound dressing.

It is reported that perforated layers may have improved properties with regard to the specific adherence requirements of the layer facing the wound. For example, US 2009/0093779 A1 describes a supple thermoplastic section of material comprising a first smooth surface; a second surface facing away from the first smooth surface and a plurality of three-dimensional perforations whose walls extend from the first smooth surface and end in an overhang with a free edge, thus making the second surface rough to touch. Said material, however, seems to be improvable with regard to the peeling properties, such as the strength of the peel force and peeling condition, once peeling (removal) is desired.

US 2008/319368 A1 relates to an adhesive skin plate wound dressing product comprising a series of perforations in a substrate material, wherein the design of the perforations can influence the peeling force. US 2019/134255 A1 relates to an adhesive tape for use in wound dressings comprising a gel adhesive, wherein the dressing is in form of a tape. US 2020/246192 A1 relates to a wound dressing with a perforated layer and an adhesive layer, wherein the perforations are arranged in a staggered manner. US 20220/323694 A1 relates to a wound dressing comprising a membrane with silicone gel adhesive coating. The membrane has perforation patterns that can be squares aligned with or at 45° from the machine direction or as equilateral triangles.

Thus, there is still a need for an adhesive material or structure which can, for example, be used for the manufacture of a wound dressing which, once applied to the skin and/or wound, remains there until its secure removal.

Hence, it was an object of the present invention to overcome the above drawbacks.

In particular, it was an object of the present invention to provide a structure which can for example be used in a wound dressing which is capable of ensuring a strength of the peel force that ensures continuance at the place the wound dressing has been applied to. In other words, it is an object of the present invention to provide a structure that prevents the wound dressing for example from slipping or even removal at an undesired time. Further, once removal is desired, the structure should also ensure safe removal without negatively affecting the surface of the skin or wound to which it has been applied.

### Summary

The present invention has unexpectedly solved the above objectives by a new perforated adhesive laminate comprising a first layer comprising a medically acceptable adhesive and a substrate, wherein the laminate has perforation holes which opening has a specific area and wherein said perforation holes form a specific perforation pattern.

Thus, the subject of the present invention is a perforated adhesive laminate comprising
(a) a first layer comprising a medically acceptable adhesive,
(b) a substrate

wherein the first layer is coated on one side of the substrate, and
   wherein the laminate has perforation holes forming a perforation pattern such that the strength of the first layer's average peel force in machine-direction and the strength of the first layer's average peel force in cross-direction differ no more than 5 % and/or the amplitude between the maximum and minimum peaks of the peel force curve are no more than 25 % of the average peel force; and
wherein the perforation holes have a surface size of between 1.75 to 12.5 mm²,
wherein the first layer is the wound contact layer,
wherein the adhesive comprised by the first layer is a silicon-based adhesive,
wherein the substrate is a film, and
wherein the sum of the areas of the openings of the perforated holes is between 10 % and 50 % of the total surface of the laminate,
wherein the perforation holes are arranged at equidistance from each other to form a square aligned to machine direction and cross direction, or
wherein the perforation holes are arranged at equidistance from each other to form a square with a 45°angle from machine direction and cross direction, or
wherein the perforation holes are arranged at equidistance from each other to form an equilateral triangle, wherein the aligned holes constitute an angle of between 10° and 20° from machine direction and cross direction.

Another aspect of the invention is an assembly comprising the perforated adhesive laminate according to the present invention and a textile or a polymer-based film.

Finally, described herein is the use of the perforated adhesive laminate according to the present invention or the assembly according to the present invention as a wound contact layer and/or backing layer of a wound dressing.

### Detailed Description of the Invention

The present perforated adhesive laminate comprises (a) a first layer comprising a medically acceptable adhesive. An adhesive can be regarded as a substance which, when applied to one or both surfaces of two distinct subjects, binds them together and resists their separation. The adhesive comprised in the first layer is medically acceptable. A medically acceptable adhesive is an adhesive which substantially does not negatively affect the environment/surface of the body to which it may be applied. Thus, when for example applied over a wound, the medically acceptable adhesive does not release inadvertently or grow together with the wound tissue, since both can be detrimental to wound healing.

Examples of medically acceptable adhesives are acrylate-based adhesives, epoxide-based adhesives, urethane-based adhesives and silicone-based adhesives. The medically acceptable adhesive comprised in the first layer is a silicon-based adhesive.

In a preferred embodiment the first layer has a thickness from 20 µm to 225 µm, more preferably from 30 µm to 215 µm, in particular from 40 µm to 200 µm.

Preferably the first layer has a thickness of about 45 µm, about 50 µm, about 60 µm, about 70 µm, about 80 µm, about 90 µm, about 100 µm, about 110 µm, about 120 µm, about 130 µm, about 140 µm, about 150 µm, about 160 µm, about 170 µm, about 180 µm or about 190 µm.

In a further preferred embodiment, the first layer (a) comprises a medically acceptable silicone-based adhesive and has a thickness of 20 µm to 225 µm, more preferably from 40 µm to 200 µm.

In a preferred embodiment the first layer consists of a medically acceptable adhesive.

Further, the present perforated adhesive laminate comprises (b) a substrate. In line with the present application a substrate can be considered as a carrier comprising an inert material. Inert materials suitable for a substrate are known in the art. Nonlimiting examples of suitable inert materials are polymers, such as polyethylene-based materials, polypropylene-based materials, polyester-based materials, such as materials from polyethylene terephthalate (PET), polyamide-based materials, polyvinylchloride-based materials, polyacrylate-based materials, polymethacrylate-based materials, polyurethane-based materials, such as materials from polyester urethane or polyether urethane, silicone-based materials and mixtures thereof. In a preferred embodiment the substrate comprises a polyurethane-based material.

The substrate is a film.

In a preferred embodiment the substrate has a thickness of 5 µm to 50 µm, more preferably 10 µm to 40 µm, in particular 20 µm to 30 µm.

Preferably the substrate has a thickness of about 21 µm, about 22 µm, about 23 µm, about 24 µm, about 25 µm, about 26 µm, about 27 µm, about 28 µm or about 29 µm, especially about 25 µm.

In a further preferred embodiment, the substrate comprises polyurethane-based material, preferably in form of a film and has a thickness of 5 µm to 50 µm, more preferably from 10 µm to 40 µm.

First layer (a) is applied on substrate (b). The laminate is construed such that the first layer (a) is the wound contact layer. In other words, the first layer is arranged such that the side opposite to the side applied on the substrate is facing the wound.

The laminate according to the present invention comprises (c) an optional second layer comprising a medically acceptable adhesive. As far as the medically acceptable adhesive comprised in optional second layer (c) is concerned, generally the same applies as described above with regard to the medically acceptable adhesive comprised in the first layer.

Thus, the medically acceptable adhesive comprised by optional second layer (c) can for example be acrylate-based adhesives, epoxide-based adhesives, urethane-based adhesives or silicone-based adhesives.

The medically acceptable adhesive comprised by optional second layer (c) can preferably be the same adhesive as the medically acceptable adhesive comprised by first layer (a).

Alternatively preferred, the medically acceptable adhesive comprised by optional second layer (c) can be another adhesive as the medically acceptable adhesive comprised by first layer (a).

It is preferred that the medically acceptable adhesive comprised in the optional second layer is an acrylate-based adhesive or a silicone-based adhesive. More preferred, the medically acceptable adhesive comprised in the optional second layer is an acrylate-based adhesive.

In a preferred embodiment the optional second layer consists of a medically acceptable adhesive.

In a preferred embodiment optional second layer (c) layer has a thickness of 5 µm to 100 µm, more preferably 10 µm to 70 µm, in particular 20 µm to 40 µm, especially about 30 µm.

Preferably the optional second layer has a thickness of about 21 µm, about 22 µm, about 23 µm, about 24 µm, about 25 µm, about 26 µm, about 27 µm, about 28 µm, about 29 µm, about 30 µm, about 31 µm, about 32 µm, about 33 µm, about 34 µm, about 35 µm, about 36 µm, about 37 µm, about 38 µm or about 39 µm.

In a further preferred embodiment, optional second layer (c) comprises a medically acceptable acrylate-based adhesive and has a thickness of 5 µm to 100 µm, in particular 20 µm to 40 µm.

In a preferred embodiment optional second layer (c) is applied onto the opposite side of the substrate onto which the first layer is applied. Thus, optional second layer (c) is optionally applied onto the wound-remote side of substrate (b).

The laminate according to the invention has perforation holes forming a perforation pattern such that the strength of the first layer's average peel force in machine-direction and the strength of the first layer's average peel force in cross-direction differ no more than 5 %, preferably no more than 4 %, in particular no more than3 %.

A perforation hole can be considered as an opening going through the perforated laminate, i.e. completely through first layer (a) and substrate (b) as well as optional second layer (c). Perforation holes can be made by any device suitable for the perforation of a laminate such as nails, needles or a punch. Alternatively, perforation can be carried out by die cutting, kiss cutting and by ultrasonic cutting.

The opening of a perforation hole can be any form. In other words, the form of the opening of a perforation hole can for example be rectangular, square, circular, elliptic, triangular, pentagonal, hexagonal, octagonal or diamond-shaped. In a preferred embodiment the openings of the perforation holes are square, circular or elliptic, preferably circular.

The openings of the perforation holes have a size of between 1.75 mm² and 12.5 mm², preferably between 2.0 mm² and 8.5 mm², in particular between 2.25 mm² and 5.0 mm².

In case that the perforation hole is circular, the opening size of a perforation hole between 1.75 mm² and 12.5 mm² substantially corresponds to a diameter of the opening hole of about 1.5 mm to about 3.9 mm. Correspondingly, again if circular, the opening size of a perforation hole between 2.25 mm² and 5.0 mm² substantially corresponds to a diameter of the opening hole of about 1.7 mm to about 2.5 mm.

The perforation holes in the laminate form a perforation pattern such that the strength of the first layer's average peel force in machine-direction and the strength of the first layer's average peel force in cross-direction differ no more than 5 %, and/or the amplitude between the maximum and minimum peaks of the peel force curve is no more than 25 %, preferably no more than 15 % of the average peel force.

The laminate can be considered to have a machine direction and a cross direction. Generally, by the process of manufacturing, such as drawing a laminate in machine-direction, said laminate can get desired properties, such as advantageous shrink properties. The cross-direction is generally a direction being inclined by a certain angle from the machine direction. Said angle is usually 0° to 90°C.

The strength of the average peel force in machine-direction and the average strength of the peel force in cross-direction and the amplitude between the maximum and minimum peaks of the peel force curve are determined as explained in the experimental section below.

By forming the perforation pattern such that the strength of the first layer's average peel force in machine-direction and the strength of the first layer's average peel force in cross-direction differ no more than 5 %, both the strength of the first layer's average peel force in machine-direction and the strength of the first layer's average peel force in cross-direction are substantially the same and thus, the known peeling off from one direction or side of the laminate can preferably be prevented or at least significantly reduced. Thus, advantageous homogenous strengths of the average peel forces of the first layer in both directions of the whole laminate can be achieved. Similar considerations apply in case that the amplitude between the maximum and minimum peaks of the peel force curve is no more than 25 % of the average peel force. Again, an advantageous homogenous peel force of the first layer and thus of the whole laminate can be achieved and peeling off the side of the laminate can be preferably prevented or at least significantly reduced.

Depending on the distance of the perforation holes from each other, the corresponding angle of distribution of said holes and the optional alignment to the machine-direction / cross direction, there are different perforation patterns.

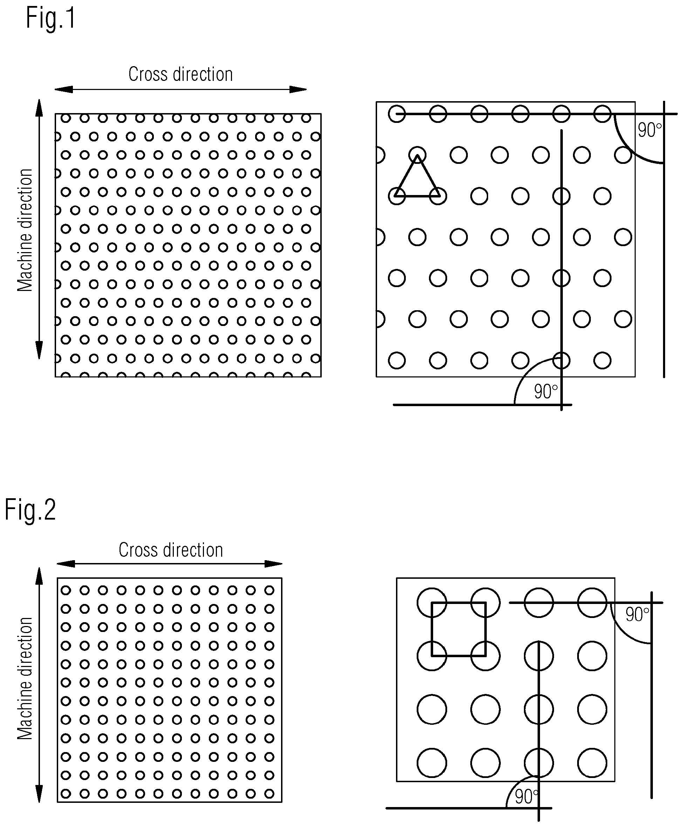

A perforation pattern with perforation holes is known, wherein the perforation holes are arranged at equidistance from each other to form an equilateral triangle (angle 60°) aligned with machine direction and cross direction is known. Such a perforation pattern can be seen in Figure 1. Due to the pattern, poor balance of the strengths of the average peel forces in machine direction and cross direction can be achieved. Further, the pattern leads to a disadvantageously high amplitude between the maximum and minimum peaks of the peel force curve.

The perforation holes are arranged at equidistance from each other to form a square aligned to machine direction and cross direction. Such a perforation pattern can be seen in Figure 2. Due to the pattern, a good balance of the strengths of the average peel forces in machine direction and cross direction can be achieved. Further, the pattern results in a high amplitude between the maximum and minimum peaks of the peel force curve.

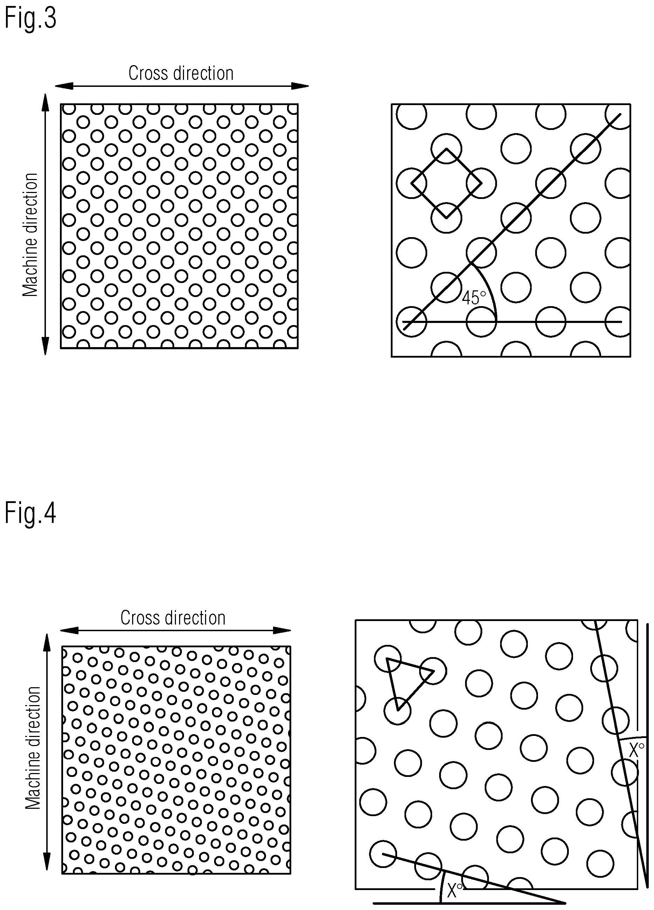

Alternatively, the perforation holes are arranged at equidistance from each other to form a square at a 45° angle from machine direction and cross direction. Such a perforation pattern can be seen in Figure 3. Due to the pattern, a good balance of the strengths of the average peel forces in machine direction and cross direction can be achieved. Further, the pattern results in a high amplitude between the maximum and minimum peaks of the peel force curve.

**In** a further, more preferred alternative the perforation holes are arranged at equidistance from each other to form an equilateral triangle (angle 60°), wherein the aligned holes constitute an angle of between 10° and 20°, preferably between 12° and 18°, more preferably between 13° and 17°, in particular about 15° from machine direction and cross direction. Such a perforation pattern with 15° from the machine and cross direction can be seen in Figure 4. Due to the pattern, a good balance of the strengths of the average peel forces in machine direction and cross direction can be achieved. Further, since there is no alternance between perforated rows and plain rows at the front of the peel direction, the pattern leads to an advantageously low amplitude between the maximum and minimum peaks of the peel force curve.

The sum of the areas of the opening of the perforated holes is between 10 % and 50 %, preferably between 12 % and 45 %, more preferably between 15 % and 30 % of the total surface of the laminate. These areas can also be referred to as open (or opened) areas of the laminate due to the absence of any material in this area.

The sum of the areas of the opening of the perforated holes is preferably about 15 %, about 16 %, about 17 %, about 18 %, about 19 %, about 20 %, about 21 %, about 22 %, about 23 %, about 24 %, about 25 %, about 26 %, about 27 %, about 28 %, about 29 % or about 30 %, more preferably about 25 %.

It turned out that through a high open area rate the present laminate exhibits good adherence to a surface such as the skin.

Further, the invention relates to an assembly comprising the perforated adhesive laminate according to the invention, wherein the laminate comprises (c) a second layer comprising a medically acceptable adhesive and wherein the second layer is applied on the opposite side of the substrate, and a textile or a polymer-based film arranged on the second layer comprised by the laminate.

In line with this application a textile can be referred to as a flexible material which can be prepared by weaving, knitting, crocheting, knotting, tatting, felting, bonding or braiding of yarns or threads and thereby creating an interlocking network of these yarns or threads. These yarns or threads in turn can be obtained by spinning raw fibres from either natural or synthetic sources into long and twisted lengths. Examples of textiles are woven fabrics, non-woven fabrics, knittings, nettings or braids, tulle or felt.

In a preferred embodiment of the assembly of the invention, the textile is a woven fabric or a non-woven fabric.

A woven fabric is a fabric made by interlacing two or more threads at right angles to one another and can for example be obtained by weaving. Woven fabrics can be produced on a loom and made of many threads woven on a warp and a weft.

A non-woven fabric is a fabric-like material which can be made from staple fibre (short) and long fibres (continuous long), bonded together by chemical, mechanical, heat or solvent treatment.

In a preferred embodiment of the invention the woven fabric or the non-woven fabric is based on natural or synthetic fibers.

Natural fibres can be referred to as fibres having a natural origin such as plants, animals or minerals. Natural fibres generally can be used without having undergone a chemical transformation reaction. Examples are silk, cellulose, cotton and wool.

Synthetic fibres can be referred to as fibres which have been either chemically synthesized or natural fibres which have been chemically transformed or modified. Examples are viscose fibres or fibres made from polymers such as poly(meth)acrylate, polyamide, polyimide, polyamidimide, polyurethane, polyester such as polyethylene terephthalate and polybutylene terephthalate, polyether, polyacrylnitrile, polyalkylene such as polyethylene and polypropylene.

In a preferred embodiment the polymer-based film comprised by the assembly has a solubility in water of less than 10 mg/l, more preferably less than 1 mg/ml, in particular from 0,0001 to 1 mg/ml; determined with column elution according to EU-Guideline RL67-548-EWG, annex V, chapter A6. The polymer-based film can, for example, comprise polymers such as polyurethane, polyether urethane, polyester urethane, polyester, polypropylene, polyethylene, polyamide, polyvinylchloride, polyorganosiloxane (silicone), polyacrylate, polymethacrylate or mixtures therefrom, preferably polyurethane.

It is preferred that the polymer-based film exhibits a water vapor permeability from at least 300 g/m²/24 hrs, in particular from at least 1000 g/m²/24 hrs and especially from at least 2000 g/m²/24 hrs to 5000 g/m²/24 hrs, determined according to DIN EN 13726, upright.

In a preferred embodiment the assembly comprises a laminate according to the invention and a textile or a polymer-based film, wherein the laminate is arranged such that the first layer of the laminate is the wound contact layer. In this case the laminate comprises a second layer comprising a medically acceptable adhesive. The textile or the polymer-based film, preferably a polymer-film, is arranged on said second layer comprised by the present laminate.

In an alternatively preferred embodiment, the assembly comprises a laminate according to the invention, a textile or a polymer-based film and a layer comprising absorbent material. An absorbent material can be referred to as a material capable of receiving and subsequently retaining a liquid such as water or exudate. Absorbent materials are known in the art. In a preferred embodiment the absorbent material is a superabsorbent material based on a copolymer of acrylic acid and acrylamide or a foam, preferably a foam based on polyurethane. The layer of the absorbent material such as a foam can preferably have a surface being smaller that the surface of the second layer comprised by the laminate. It is further preferred that the layer of absorbent material is arranged on or adhered to the centre of the second layer comprised by the laminate, such that the four rims of the second layer are formed which are not covered by the layer of absorbent material and which have substantially of the same area size. In a preferred embodiment the textile or a polymer-based film can be adhered to the medically acceptable adhesive of said rims of the second layer comprised by the laminate. Put differently, the first layer of the present laminate can be used as wound contact layer, while the second layer comprised by the laminate enables the formation of an assembly optionally having further layers.

Moreover, the invention is related to a wound care product comprising the perforated adhesive laminate according to the invention and/or the assembly according to the invention. As far as the perforated adhesive laminate and/or the assembly are concerned, it applies substantially the same as described above. Preferably the wound care product is a wound dressing.

Further, described herein is the use of the perforated adhesive laminate according to the invention as a wound contact layer or as a backing layer of a wound dressing. It is preferred that the first layer comprised by perforated adhesive laminate according to the invention is used as a wound contact layer of a wound dressing. The properties of the layer ensure an excellent adherence to the surface of the skin and enable a safe and easy removal without negatively affecting the surface of the skin or wound to which it has been applied.

As described herein, the perforated adhesive laminate according to the invention is used as a wound contact layer of an island-like wound dressing. As far as the first layer comprised by the laminate is concerned, the same as above applies. The island-like wound dressing further preferably comprises a laminate having a second layer comprising a medically acceptable adhesive on which a layer of absorbent material is adhered. With refence to the above the surface of the layer comprising the absorbent material is preferably smaller than the surface of the second layer comprised by the laminate and preferably is arranged on or adhered to the centre of the second layer comprised by the laminate, such that the four rims of the second layer are formed. These rims which are not covered by the layer of absorbent material preferably have substantially the same area size. In a preferred embodiment the textile or a polymer-based film can be adhered to the medically acceptable adhesive of said rims of the second layer comprised by the laminate. In other words, the first layer comprised by the present laminate can be used as the wound contact layer, while the second layer comprised by the laminate enables the formation of an assembly optionally having further layers.

As alternatively described herein, the perforated adhesive laminate according to the invention can be used as a backing layer of a wound dressing, in particular of a wound dressing having a pad, preferably a pad comprising an absorbent material, wherein the pad is directly into contact with the wound. It is preferred that the pad, preferably the pad comprising an absorbent material, is of such a size that the four rims of the first layer overlap the pad thus adhere to the surface, preferably the skin, surrounding the pad. It is further preferred that the perforated adhesive laminate does not comprise a second layer comprising a medically acceptable adhesive. In other words, the substrate of the perforated adhesive laminate can be considered as the backing layer of a wound dressing.

As alternatively described herein, the assembly according to the invention can be used as a backing layer of a wound dressing, in particular a wound dressing having a pad, preferably a pad comprising an absorbent material, wherein the pad is directly in contact with the wound. It is preferred that the pad, preferably the pad comprising an absorbent material, is of such a size that the four rims of the first layer overlap the pad to adhere to the surface, preferably the skin, surrounding the pad. It is further preferred that the assembly comprises a laminate comprising layer (a), (b) and (c) as well as and a textile or a polymer-based film, wherein the textile or a polymer-based film is arranged on or adhered to (c) the second layer comprising a medically acceptable adhesive. In other words, the textile or the polymer-based film of the assembly can be considered as a backing layer of a wound dressing.

### Experimental Part:

### 1. Orientation of the perforation pattern

Laminate:

| | |
|---|---|
| Layer (a): | Silicone adhesive with a thickness of 120 µm |
| Substrate (b) | Polyurethane film with a thickness of 25 µm |
| Layer (c): | Acrylic adhesive with a thickness of 30 µm |

Four laminates of about 175 x 175 mm are perforated with holes having a diameter of 2 mm with a known perforation method, ultrasonic cutting, such that four different perforation patterns each having an area of the openings of the perforated holes of about 25 % of the total surface of the laminate are obtained. These patterns are shown in Figures 1 to 4.

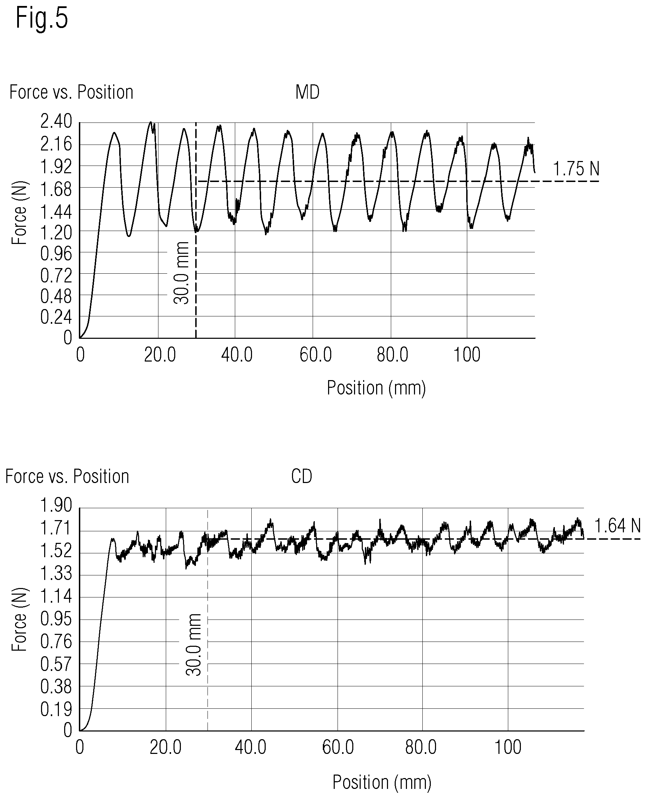

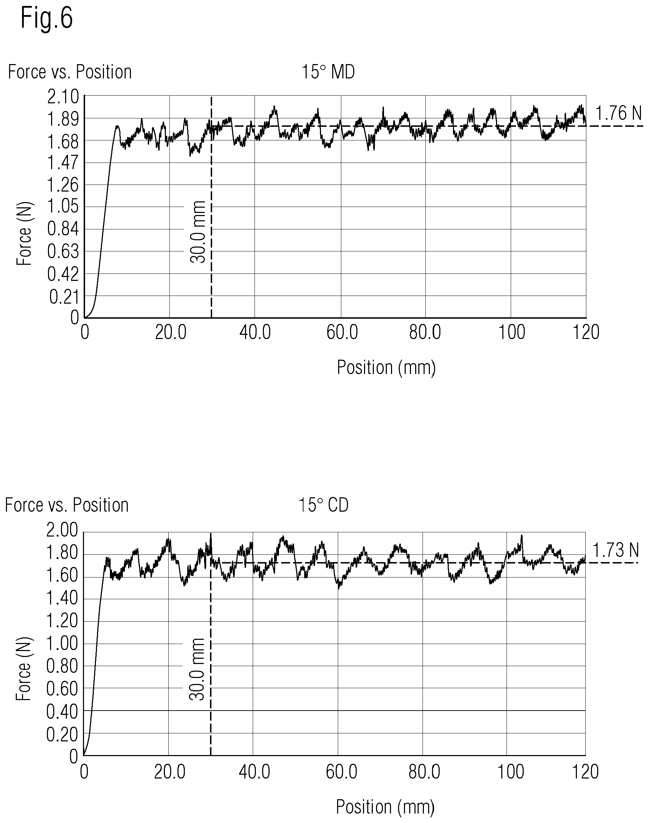

The peel force on Bristol paper of a laminate having a pattern according to Figure 1 and a pattern according to Figure 4 has been determined and is shown in Figures 5 and 6, wherein the peel force has been determined by a method based on FINAT n°1. The peel force is determined over a width of 25 mm. The characteristic values are shown Table 1 (lines with 0° MD, 0° CD correspond to a pattern according to Figure 1; lines with 15° MD, 15° CD correspond to a pattern according to Figure 4).

**Table 1:**

| tilt | peaks average [N] | valleys average [N] | peel force average [N] | amplitude of peaks/valleys | amplitude/ peel force [%] |
|---|---|---|---|---|---|
| 0° MD | 2.24 | 1.22 | 1.75 | 1.02 | 58 |
| 0° CD | 1.78 | 1.52 | 1.64 | 0.26 | 16 |
| - | - | - | - | - | - |
| 15° MD | 1.86 | 1.52 | 1.76 | 0.34 | 20 |
| 15° CD | 1.85 | 1.52 | 1.73 | 0.33 | 20 |

| | | | | | |
|---|---|---|---|---|---|
| MD: machine direction CD: cross direction | | | | | |

As can be seen from that table, a perforation pattern according to Figure 4 (tilt 15°) has a strength of average peel force in machine-direction and a strength of average peel force in cross-direction which differ less than 2 %. Moreover, the amplitude between the maximum and minimum peaks of the peel force curve is 20 % of the average peel force.

### 2. Properties of an assembly comprising the perforated adhesive laminate and a polymer-based film

Laminate:

| | |
|---|---|
| Layer (a): | Silicone adhesive with a thickness of 150 µm |
| Substrate (b) | Polyurethane film with a thickness of 25 µm |
| Layer (c): | Acrylic adhesive with a thickness of 30 µm |

Nine laminates of about 175 x 175 mm are perforated each with holes of a specific diameter with a known perforation method, ultrasonic cutting, such that each laminate has a perforation pattern according to Figure 4. Subsequently a polyurethane film of 15 µm is adhered to the second layer (c) of each laminate. The characteristic parameters of the samples are shown in Table 2.

**Table 2:**

| Sample | Diameter of the perforation hole [mm] | Number of perforation holes per cm² | Open area [%] |
|---|---|---|---|
| 1 | 1.3 | 12.4 | 16.5 |
| 2 | 1.4 | 12.4 | 19.1 |
| 3 | 1.5 | 12.4 | 21.9 |
| 4 | 1.6 | 12.4 | 25 |
| 5 | 1.7 | 12.4 | 28.2 |
| 6 | 1.7 | 11.0 | 25 |
| 7 | 1.8 | 9.8 | 25 |
| 8 | 1.9 | 8.8 | 25 |
| 9 | 2.0 | 7.9 | 25 |

| | | | |
|---|---|---|---|
| Open area: sum of the areas of the openings of the perforated holes divided by the total surface of the laminate | | | |

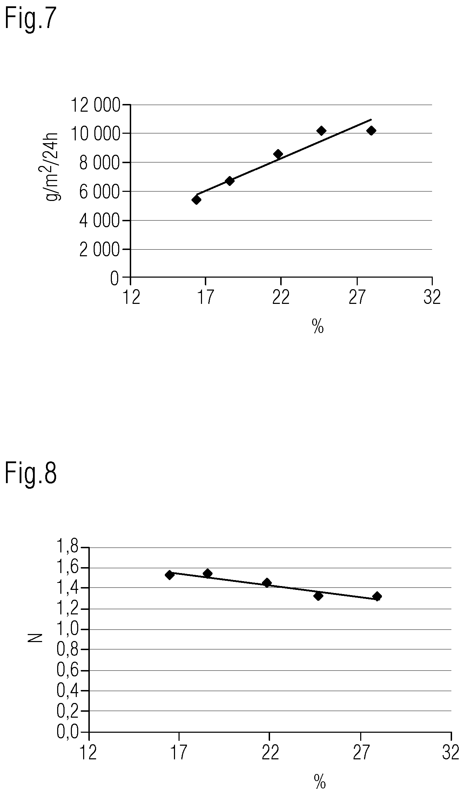

Further, the moisture vapor transmission rates (determined by DIN EN 13726-2:2002, upright) of samples 1, 2, 3, 4 and 5 has been determined. A curve showing the values of the moisture vapor transmission rates in "g/m²/24h" (Y-axis) and the corresponding values of the open area in "%" (X-axis) has been prepared. The corresponding diagram is shown in Figure 7.

Further, the average peel force of samples 1, 2, 3, 4 and 5 has been determined. A curve showing the values of the average peel force in "N" (Y-axis) and the corresponding values of the open area in % (X-axis) has been prepared. The corresponding diagram is shown in Figure 8.

Peel force and the moisture vapor transmission rate are apparent antinomic properties. Moisture vapor transmission rate rises with an increasing open area while the peel force decreases with an increasing open area (see Figures 7 and 8).

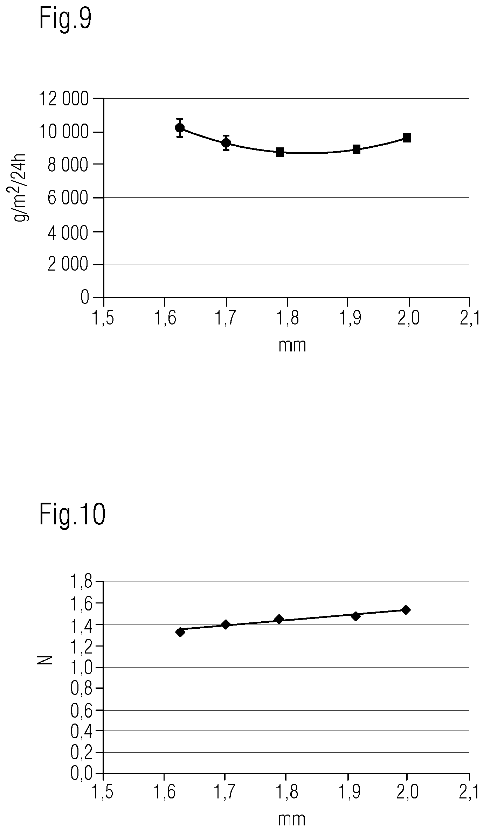

Further, the moisture vapor transmission rates (determined by DIN EN 13726-2:2002, upright) of samples 4, 6, 7, 8 and 9 (all having an open area of 25 %) have been determined. A curve showing the values of the moisture vapor transmission rates in "g/m²/24h" (Y-axis) and the corresponding values of the diameter of the opening of the perforation holes in "mm" (X-axis) has been prepared. The corresponding diagram is shown in Figure 9.

Further, the average peel force of samples 4, 6, 7, 8 and 9 (all having an open area of 25 %) has been determined. A curve showing the values of the average peel force in "N" (Y-axis) and the corresponding values of the diameter of the opening of the perforation holes in "mm" (X-axis) has been prepared. The corresponding diagram is shown in Figure 10.

From Figures 9 and 10 it can be derived that, taking a fixed open area, the diameter of the perforation holes plays an important role for the corresponding average peel force and the moisture vapor transmission rate of the assembly. In summary, taken a fixed open area, bigger perforation holes would result in a good peel force as well as in a good moisture vapor transmission rate.

## Claims

1. Perforated adhesive laminate comprising
(a) a first layer comprising a medically acceptable adhesive,
(b) a substrate
wherein the first layer is applied on one side of the substrate; and
wherein the laminate has perforation holes forming a perforation pattern such that the strength of the first layer's average peel force in machine-direction and the strength of the first layer's average peel force in cross-direction differ no more than 5 % and/or the amplitude between the maximum and minimum peaks of the peel force curve are no more than 25 % of the average peel force; and
wherein the openings of the perforation holes have an area of between 1.75 mm² to 12.5 mm²
wherein the first layer is the wound contact layer,
wherein the adhesive comprised by the first layer is a silicon-based adhesive,
wherein the substrate is a film, and
wherein the sum of the areas of the openings of the perforated holes is between 10 % and 50 % of the total surface of the laminate,
wherein the perforation holes are arranged at equidistance from each other to form a square aligned to machine direction and cross direction, or
wherein the perforation holes are arranged at equidistance from each other to form a square with a 45°angle from machine direction and cross direction, or
wherein the perforation holes are arranged at equidistance from each other to form an equilateral triangle, wherein the aligned holes constitute an angle of between 10° and 20° from machine direction and cross direction.

2. Perforated adhesive laminate according to claim 1, wherein the openings of the perforated holes have an area between 2.25 mm² and 5.0 mm².

3. Assembly comprising the perforated adhesive laminate according to any one of the preceding claims, wherein the laminate comprises (c) a second layer comprising a medically acceptable adhesive and wherein the second layer is applied on the opposite side of the substrate, and a textile or a polymer-based film arranged on the second layer comprised by the laminate.

4. Assembly according to claim 3, wherein the textile is a woven or nonwoven fabric, preferably a woven or nonwoven fabric based on natural or synthetic fibers.

5. Assembly according to claim 3, wherein the polymer-based film is based on polyethylene, polypropylene, polyurethane or copolymers or blends thereof.

6. Assembly according to any of claims 3 to 5, wherein the adhesive comprised by the second layer is an acrylate-based adhesive.

7. Wound care product comprising the perforated adhesive laminate according to any one of claims 1 to 2 or the assembly according to any one of claims 3 to 6.

## Patentansprüche

1. Perforiertes Klebelaminat, umfassend
(a) eine erste Schicht, die einen medizinisch verträglichen Klebstoff umfasst,
(b) ein Substrat,
wobei die erste Schicht auf einer Seite des Substrats aufgebracht ist; und
wobei das Laminat Perforationslöcher aufweist, die ein Perforationsmuster bilden, so dass sich die Stärke der durchschnittlichen Schälkraft der ersten Schicht in Maschinenrichtung und die Stärke der durchschnittlichen Schälkraft der ersten Schicht in Querrichtung um nicht mehr als 5 % unterscheiden und/oder die Amplitude zwischen den maximalen und minimalen Spitzen der Schälkraftkurve nicht mehr als 25 % der durchschnittlichen Schälkraft beträgt; und
wobei die Öffnungen der Perforationslöcher eine Fläche zwischen 1,75 mm² und 12,5 mm² aufweisen,
wobei die erste Schicht die Wundkontaktschicht ist,
wobei der Klebstoff, der von der ersten Schicht umfasst wird, ein Klebstoff auf Silikonbasis ist,
wobei das Substrat ein Film ist, und
wobei die Summe der Flächen der Öffnungen der Perforationslöcher zwischen 10 % und 50 % der Gesamtoberfläche des Laminats beträgt,
wobei die Perforationslöcher in gleichem Abstand voneinander angeordnet sind, um ein Quadrat zu bilden, das in Maschinenrichtung und Querrichtung ausgerichtet ist, oder
wobei die Perforationslöcher in gleichem Abstand voneinander angeordnet sind, um ein Quadrat mit einem 45°-Winkel von Maschinenrichtung und Querrichtung zu bilden, oder
wobei die Perforationslöcher in gleichem Abstand voneinander angeordnet sind, um ein gleichseitiges Dreieck zu bilden, wobei die ausgerichteten Löcher einen Winkel zwischen 10° und 20° von Maschinenrichtung und Querrichtung bilden.

2. Perforiertes Klebelaminat nach Anspruch 1, wobei die Öffnungen der Perforationslöcher eine Fläche zwischen 2,25 mm² und 5,0 mm² aufweisen.

3. Anordnung, umfassend das perforierte Klebelaminat nach einem der vorhergehenden Ansprüche, wobei das Laminat (c) eine zweite Schicht umfasst, die einen medizinisch verträglichen Klebstoff umfasst, und wobei die zweite Schicht auf der gegenüberliegenden Seite des Substrats aufgebracht ist, und ein Textil oder einen Film auf Polymerbasis, der auf der zweiten Schicht angeordnet ist, die von dem Laminat umfasst wird.

4. Anordnung nach Anspruch 3, wobei das Textil ein gewebter oder nicht gewebter Stoff ist, vorzugsweise ein gewebter oder nicht gewebter Stoff auf Basis von natürlichen oder synthetischen Fasern.

5. Anordnung nach Anspruch 3, wobei der Film auf Polymerbasis auf Polyethylen, Polypropylen, Polyurethan oder Copolymeren oder Mischungen davon basiert.

6. Anordnung nach einem der Ansprüche 3 bis 5, wobei der Klebstoff, der von der zweiten Schicht umfasst wird, ein Klebstoff auf Acrylatbasis ist.

7. Wundversorgungsprodukt, umfassend das perforierte Klebelaminat nach einem der Ansprüche 1 bis 2 oder die Anordnung nach einem der Ansprüche 3 bis 6.

## Revendications

1. Laminé adhésif perforé comportant :
a) une première couche comportant un adhésif acceptable du point de vue médical ;
b) un substrat,
dans lequel la première couche est appliquée sur un côté du substrat ; et
dans lequel le laminé a des trous de perforation formant un motif de perforation de telle sorte que la résistance à la force de pelage moyenne de la première couche dans un sens machine et la résistance à la force de pelage moyenne de la première couche dans un sens travers ne diffèrent pas de plus de 5 % et/ou l'amplitude entre les pics maximum et minimum de la courbe de force de pelage n'est pas supérieure à 25 % de la force de pelage moyenne ; et
dans lequel les ouvertures des trous perforés ont une aire comprise entre 1,75 mm² et 12,5 mm²,
dans lequel la première couche est la couche en contact avec la plaie,
dans lequel l'adhésif compris dans la première couche est un adhésif à base de silicone,
dans lequel le substrat est un film, et
dans lequel la somme des aires des ouvertures des trous perforés est comprise entre 10 % et 50 % de la surface totale du laminé,
dans lequel les trous de perforation sont disposés à équidistance les uns des autres pour former un carré aligné dans le sens machine et dans le sens travers, ou
dans lequel les trous de perforation sont disposés à équidistance les uns des autres pour former un carré avec un angle de 45° par rapport au sens machine et au sens travers, ou
dans lequel les trous de perforation sont disposés à équidistance les uns des autres pour former un triangle équilatéral, dans lequel les trous alignés constituent un angle compris entre 10° et 20° par rapport au sens machine et au sens travers.

2. Laminé adhésif perforé selon la revendication 1, dans lequel les ouvertures des trous perforés ont une aire comprise entre 2,25 mm² et 5,0 mm².

3. Ensemble comportant le laminé adhésif perforé selon l'une quelconque des revendications précédentes, dans lequel le laminé comporte (c) une seconde couche comportant un adhésif acceptable du point de vue médical et dans lequel la seconde couche est appliquée sur le côté opposé du substrat, et un textile ou un film à base de polymère disposé sur la seconde couche comprise dans le laminé.

4. Ensemble selon la revendication 3, dans lequel le textile est un tissu ou un non-tissé, de préférence un tissu ou un non-tissé à base de fibres naturelles ou synthétiques.

5. Ensemble selon la revendication 3, dans lequel le film à base de polymère est à base de polyéthylène, de polypropylène, de polyuréthane ou de copolymères ou mélanges de ceux-ci.

6. Ensemble selon l'une quelconque des revendications 3 à 5, dans lequel l'adhésif compris dans la seconde couche est un adhésif à base d'acrylate.

7. Produit de soin de plaie comportant le laminé adhésif perforé selon l'une quelconque des revendications 1 à 2 ou l'ensemble selon l'une quelconque des revendications 3 à 6.
